# EUROPEAN PATENT APPLICATION

(11) **EP 3 929 300 A1**
(43) Date of publication of application: **29.12.2021**
(21) Application number: 20182737.5
(22) Date of filing: 26.06.2020
(51) Int. Cl.: C12P 19/04, C12P 19/18, C07K 14/245, C07K 14/255, C07K 14/26, C07K 14/195, C07K 14/36, C07K 14/21

(54) **IMPROVED EXPORT OF OLIGOSACCHARIDES FROM BACTERIAL CELLS**

(71) Applicant: Chr. Hansen HMO GmbH, 53619 Rheinbreitbach (DE)
(72) Inventor: ENGLERT, Markus, 53604 Bad Honnef (DE); JENNEWEIN, Stefan, 53605 Bad Honnef (DE); FRERIGMANN, Henning, 50676 Köln (DE); PARSCHAT, Katja, 53173 Bonn (DE); TRÖTSCHEL, Christian, 50767 Köln (DE)

(57) **Abstract**

Disclosed are genetically-engineered gram-negative bacterial cells for the production of an oligosaccharide of interest, wherein the bacterial cell possesses a saccharide transporter and/or a porin being expressed from a recombinant gene or a deregulated endogenous gene, thereby facilitating the translocation of the oligosaccharide of interest from the cell's cytoplasm into the cell's environment. Also disclosed are methods for producing an oligosaccharide of interest which used said genetically-engineered gram-negative bacterial cells.

## Description

The present invention relates to the production of oligosaccharides in bacteria, and to bacteria for the production of oligosaccharides.

### Background

Oligosaccharides are saccharide molecules consisting of a small number of monosaccharide moieties which are linked to one another by glycosidic bonds. Oligosaccharides are of particular interest in medicine and nutrition, because they have many functions including energy storage, cell recognition and cell adhesion. In addition, oligosaccharides can serve as prebiotics.

Oligosaccharides may be present as glycans being attached to proteins or lipids. Examples of naturally occurring oligosaccharides that are not a component of glycol-proteins or glycolipids are maltodextrins which result from the microbial breakdown of starch. Another example are osmo-regulated oligosaccharides which are produced by microorganisms in response to osmotic stress.

Some oligosaccharides are non-digestible oligosaccharides, i.e. oligosaccharides that can not be digested in the human small intestine. These non-digestible oligosaccharides gained a lot of interest lately as they may have prebiotic effects. Thus, they pass through the large intestine, where they promote growth of *Bifidobacteria,* which are regarded as beneficial for the alimentary system. A subset of these dietary oligosaccharides, the so-called human milk oligosaccharides (HMOs) are of particular interest as they are exclusively found in human milk, and were identified to contribute to an infants' development of a healthy microbiome, immune system and its cognitive capabilities.

As only some HMOs are found in natural sources others than human breast milk, e.g. the most dominant human milk oligosaccharide 2'-fucosyllactose was found in the milk of minke whales, African lions, polar bears, bonobos, chimpanzees and orang-utans, but not in the milk of domesticated dairy animals, it requires their production - rather than isolation from natural sources - to make them available as ingredients for nutritional compositions including, but not limited to - infant formula. Theoretically, oligosaccharides may be produced by chemical synthesis or by biocatalysis *in vitro.* Chemical synthesis of oligosaccharides involves stepwise addition of monosaccharide moieties at the reducing or non-reducing end of a saccharide chain, which addition included introduction and removal of protective groups. Further considering the peculiarities of stereochemistry, it turned out that chemical synthesis is not suitable for providing oligosaccharides in an industrial scale at reasonable costs to the food industry. Alternatively, oligosaccharides may be produced by biochemical reactions in that enzymes catalyze the formation of glycosidic bonds between saccharide moieties *in vitro.* However, biocatalysis is also not suitable for an industrial scale manufacturing of oligosaccharides at reasonable costs such that the oligosaccharides may be used in nutritional compositions.

To overcome the drawbacks of chemical and biochemical production of oligosaccharides, fermentative production processes for some oligosaccharides were developed in recent years using recombinant *E. coli* cells. In these processes, genetically engineered *E*. *coli* cells being able to intracellularly synthesize an oligosaccharide of interest are cultivated in a culture medium and under conditions that are permissive for the *E. coli* cell to synthesize the oligosaccharide of interest. At the end of the fermentation step, the oligosaccharide of interest is recovered from the culture medium and/or the *E. coli* cells.

It is advantageous to recover an oligosaccharide of interest from the culture medium rather than from the bacterial cells, because the former recovery will involve less process steps, is less costly and cumbersome than the recovery from a cell lysate. In addition, it is beneficial to have an oligosaccharide of interest to be exported from the bacterial cell into the culture medium, because intracellular accumulation of the oligosaccharide of interest poses an osmotic stress to the bacterial cell and may thus affect productivity. It is therefore desired to induce or enhance export of an oligosaccharide of interest that is synthesized intracellularly by a microbial production strain from the cell's cytoplasm into the culture medium said bacterial cell is cultivated in.

It has surprisingly been found that recombinant expression of a saccharide transporter and/or a porin in a gram-negative bacterial cell that is able to synthesize an oligosaccharide of interest mediates or at least facilitates translocation of the desired oligosaccharide from the cell's cytoplasm into the culture medium.

### Summary

In a first aspect, provided are genetically-engineered gram-negative bacterial cells for the production of an oligosaccharide of interest, wherein said bacterial cells are able to intracellularly synthesize the oligosaccharide of interest, and possess a saccharide transporter in their inner membrane and/or a porin in their outer membrane for the translocation of the oligosaccharide of interest from the cells' cytoplasm into the culture medium, wherein the saccharide transporter and/or the porin is expressed from a recombinant gene or a deregulated endogenous gene.

In a second aspect, provided is the use of a genetically-engineered gram-negative bacterial cell being able to intracellularly synthesize an oligosaccharide of interest, and possessing a saccharide transporter in its inner membrane and/or a porin in its outer membrane for translocating of the oligosaccharide of interest from the cell's cytoplasm into the culture medium, wherein the saccharide transporter and/or the porin is expressed from a recombinant gene or a deregulated endogenous gene, for the production of an oligosaccharide of interest.

In a third aspect, provided are methods for the production of an oligosaccharide of interest, wherein the oligosaccharide of interest is synthesized in a genetically engineered gram-negative bacterial cell according to the first aspect, is translocated from the bacterial cell's cytoplasm across the inner membrane and the outer membrane of the bacterial cell into the culture medium, and is recovered from the culture medium.

In a further aspect, provided is a method for enhancing the translocation of an oligosaccharide of interest that is produced in a genetically-engineered gram-negative bacterial cell across the inner membrane and the outer membrane of said gram-negative bacterial cell into the culture medium.

### Brief description of the drawings

- FIG. 1: displays a column graph showing the relative amount of 3-FL in the culture medium of bacterial cells expressing a porin from a recombinant gene.
- FIG. 2: displays a column graph showing the relative expression level of the saccharide transporter MdfA in two *E. coli* strains.
- FIG. 3: displays a column graph showing the ratio of LNT-II or LNT in the culture medium vs. cytoplasm of *E. coli* cells expressing the saccharide transporter MdfA at different levels.
- FIG. 4: displays a column graph showing the ratio of LNT-II or LNT in the culture medium vs. cytoplasm of *E. coli* cells expressing the saccharide transporter MdfA and different porins.
- FIG. 5: displays a column graph showing the relative extracellular amounts of LNnT produced by *E. coli* cells possessing a saccharide transporter and/or a porin.
- FIG. 6: displays a column graph showing the relative amount of extracellular LNnT produced by *E. coli* cells possessing a saccharide transporter and different porins.
- FIG. 7: displays a column graph showing the relative amounts of extracellular LNnT produced by *E. coli* cells possessing a porin and different saccharide transporters.
- FIG. 8: displays a column graph showing the amount of 6'-SL in the culture medium of *E. coli* cells possessing different porins.
- FIG. 9: displays a column graph showing the amount of 6'-SL in the culture medium of *E. coli* cells possessing different combinations of a saccharide transporter and a porin.

### Detailed description

According to the first aspect, provided is a genetically-engineered gram-negative bacterial cell for the production of an oligosaccharide of interest, wherein the bacterial cell is able to synthesize the oligosaccharide of interest intracellularly, and possesses a saccharide transporter in its inner membrane for translocating the oligosaccharide of interest from the cytoplasm across the inner membrane into the periplasmic space, and further possesses a porin in its outer membrane for translocating the oligosaccharide of interest from the periplasmic space across the outer membrane into the environment of the bacterial cell, e.g. the culture medium. Either the saccharide transporter or the porin or both, the saccharide transporter and the porin is/are expressed from a recombinant gene or from a deregulated endogenous gene. Expression of the saccharide transporter and/or the porin from a recombinant gene enables expression of a heterologous saccharide transporter and/or a heterologous porin, while expression of the saccharide transporter and/or the porin from a deregulated endogenous gene enables overexpression of an endogenous saccharide transporter and/or an endogenous porin during fermentative production of the oligosaccharide of interest.

The genetically-engineered bacterial cell for the production of the oligosaccharide of interest is a gram-negative bacterial cell. Gram-negative bacteria are bacteria that do not retain the crystal violet stain used in Gram staining for differentiation of bacteria. Gram-negative bacteria are characterized by their cell envelope, which is composed of a thin peptidoglycan cell wall that is sandwiched between an inner cell membrane, also called "inner membrane" or "cytoplasmic membrane" or "cell membrane", and an outer membrane. Said peptidoglycan wall between the inner membrane and the outer membrane is also designated as periplasmic space.

Examples of gram-negative bacteria that may be used for the production of the oligosaccharide of interest are bacterial strains from the genera *Pseudomonas* and *Escherichia.* In some embodiments, the gram-negative bacterial cell of the production of an oligosaccharide of interest is a strain of *Escherichia coli* such as a descendent of the K strain or the B strain of *E. coli.*

The genetically-engineered gram-negative bacterial cell is able to synthesize the oligosaccharide of interest. The term "oligosaccharide" as used herein defines a group of carbohydrates bearing a large number of structurally distinct compounds. The term "oligosaccharide" refers to a polymeric saccharide molecule consisting of at least three monosaccharide moieties, but of no more than 12 monosaccharide moieties, preferably of no more than 10 monosaccharide moieties, that are linked to one another by glycosidic bonds. Hence, the oligosaccharide of interest may consist of three, four, five, six, seven, eight, nine, ten, eleven or twelve monosaccharide moieties. The oligosaccharide of interest may consist of a linear chain of monosaccharide moieties, or it may consist as a branched chain of monosaccharide moieties, wherein at least one monosaccharide moiety has at least three monosaccharide moieties bound to it by glycosidic bonds. The monosaccharide moieties of the oligosaccharide of interest may be selected from the group consisting of aldoses (e.g. arabinose, xylose, ribose, desoxyribose, lyxose, glucose, idose, galactose, talose, allose, altrose, mannose, etc.), ketoses (e.g. ribulose, xylulose, fructose, sorbose, tagatose, etc.), deoxysugars (e.g. rhamnose, fucose, quinovose, etc.), deoxy-aminosugars (e.g. *N*-acetylglucosamine, *N*-acetyl-mannosamine, *N*-acetyl-galactosamine etc.), uronic acids (e.g. galacturonsäure, glucuronsäure etc.) and ketoaldonic acids (e. g. *N*-acetylneuraminic acid).

In one embodiment the oligosaccharide of interest is selected from the group consisting of neutral oligosaccharides, sialylated oligosaccharides and *N*-acetyl-lactosamines, i.e. oligosaccharides comprising at least one *N*-acetyl-glucosamine moiety. In an additional and/or alternative embodiment the oligosaccharide or interest is selected from the group consisting of fucosylated oligosaccharides, i.e. oligosaccharides comprising at least one fucose moiety. In an additional and/or alternative embodiment the oligosaccharide of interest is selected from the group consisting of acidic sugar, i.e. oligosaccharides comprising at least one *N*-acetylneuraminic acid moiety.

In certain embodiments the oligosaccharide of interest is a human milk oligosaccharide. In an additional and/or alternative embodiment, the oligosaccharide of interest is selected from the group consisting of 2'-fucosyllactose (2'-FL), 3-fucosyllactose (3-FL), 6'-fucosyllactose (6'-FL), 3'-galactosyllactose (3'-GL), 4'-galactosyllactose (4'-GL), 6'-galactosyllactose (6'-GL), 3'-sialyllactose (3-SL), 6'-sialyllactose (6-SL), 2',3-di-fucosyllactose (DiFL), lacto-*N*-triose II (LNT II), lacto-*N*-neotetraose (LNnT), lacto-*N*-tetraose (LNT), lacto-*N*-fucopentaose I (LNFP I), lacto-*N*-fucopentaose II (LNFP II), lacto-*N*-fucopentaose III (LNFP III), lacto-*N*-fucopentaose V (LNFP IV), lacto-*N*-hexaose, lacto-*N*-neohexaose, lacto-*N*-difucosylhexaose I, lacto-*N*-difucosylhexaose II, lacto-*N*-difucosylhexaose III, 3-fucosyl-3'-sialyllactose, sialyllacto-*N-*tetraose a, sialyllacto-*N*-tetraose b, sialyllacto-*N*-tetraose c, disialyl-lacto-*N*-tetraose, human blood group antigen A type 1, human blood group B typ 1, human blood group antigen A type 2, or human blood group antigen B type 2.

The genetically-engineered gram-negative bacterial cell is able to produce the oligosaccharide saccharide by its intracellular biosynthesis. Thus, the genetically-engineered gram-negative bacterial cell possesses the enzymes that are necessary to catalyze the conversion of an acceptor molecule to the oligosaccharide of interest by transferring a monosaccharide moiety from a donor substrate to the acceptor molecule.

In some embodiments, the acceptor molecule is a disaccharide that is converted into a trisaccharide. In other embodiments, the acceptor molecule is a trisaccharide that is converted into a tetrasaccharide. In other embodiments, the acceptor molecule is a tetrasaccharide that is converted into a pentasaccharide. In some embodiments, the acceptor molecule is a pentasaccharide that is converted into a hexasaccharide. The acceptor molecule may be internalized by the bacterial cell, preferably by utilizing a specific importer. Additionally and/or alternatively the acceptor molecule can be synthesized by the bacterial cell intracellularly.

In particular embodiments, the acceptor substrate is a monosaccharide that is internalized by the bacterial cell and is elongated by a monosaccharide moiety to obtain a disaccharide, wherein said disaccharide is further elongated by the addition of further monosaccharide moieties such that and oligosaccharide of interest is synthesized which consists of three, four, five, six or more monosaccharide moieties. For example, the bacterial cell internalizes glucose which is converted to lactose by the addition of a galactose moiety. Said lactose may then be converted to an oligosaccharide of interest such as, e.g., 2'-FL, 3-FL, LNT-II, LNT, LNnT, 3'-SL, 6'-SL or any other human milk oligosaccharide.

The gram-negative bacterial cell may have been genetically engineered to possess the enzymes mediating the conversion of an acceptor molecule, i.e. a monosaccharide, a disaccharide or an oligosaccharide to the oligosaccharide of interest by transferring a monosaccharide moiety from a donor substrate to the acceptor molecule. Hence, the gram-negative bacterial cell may contain at least one recombinant gene which encodes an enzyme mediating the conversion of the acceptor molecule to a disaccharide or oligosaccharide of interest.

Genes or equivalent functional nucleic acid sequences conferring the enzymatic capability to the gram-negative bacterial cell to add a monosaccharide moiety to an acceptor molecule that has been internalized or synthesized intracellularly can be homologous nucleic acid sequences or heterologous nucleic acid sequences. Heterologous nucleic acid sequences may originate from a plants, an animal, a bacterium, an archaea, a fungus or a virus.

The enzyme catalyzing the transfer a monosaccharide moiety to the acceptor molecule is a glycosyltransferase or a transglycosidase. In some embodiments the glycosyltransferase catalyzes the transfer of a monosaccharide moiety from a nucleotide activated sugar as donor substrate to the acceptor. The glycosyltransferase may be selected from the group consisting of galactosyltransferases, glucosamyltransferases, sialyltransferases, *N*-acetylglucosaminyltransferases, *N*-acetylgalactosaminyltransferases, glucuronosyl transferases, mannosyl transferases, xylosyl transferases, and fucosyltransferases. In an additional and/or alternative embodiment, the glycosyltransferase is selected from the group consisting of α-1,2-mannosyltransferases, β-1,4-xylosyltransferases, β-1,3-*N-*acetylglucosaminyltransferases, β-1,6-*N*-acetylglucosaminyltransferases, β-1,3-*N-*acetylgalactosaminyltransferases, β-1,4-*N*-acetylgalactosaminyltransferases, α-1,3-*N*-acetylgalactosaminyltransferases, β-1,3-galactosyltransferases, β-1,4-galactosyltransferases, β-1,6-galactosyltransferases, α-1,3-glucosyltransferases, α-1,4-glucosyltransferases, α-2,3-sialyltransferases, α-2,6-sialyltansferases, α-2,8-sialyltansferases, α-1,2-fucosyltransferases and α-1,3/1,4-fucosyltransferases.

In some embodiments the donor substrate for the monosaccharide moiety is a nucleotide-activated sugar. Typically, nucleotide-activated sugars are used by glycosyltransferases as donor substrate for the monosaccharide moiety. The nucleotide-activated sugar may be selected from the group consisting of GDP-L-fucose, UDP-galactose, UDP-glucose, CMP-*N*-acetylneuraminic acid, GDP-mannose, UDP-*N*-acetylgalactosamine, UDP-*N*-acetylglucosamine. It is understood that a fucosyltransferase uses GDP-L-fucose as donor substrate while a sialyltransferase uses CMP-*N*-acetylneuraminic acid as donor substrate, and so on.

The nucleotide-activated sugar can be synthesized by the genetically engineered bacterial cell in a *de novo* pathway. Additionally and/or alternatively, the bacterial cell uses a salvage pathway for providing the nucleotide-activated sugar.

In embodiments wherein the donor substrate is synthesized by a *de novo* pathway, the bacterial cell possesses the enzymes that are necessary for the *de novo* biosynthesis of the nucleotide-activated sugar. In some embodiments, the bacterial cell has been genetically engineered to possess at least one recombinant gene encoding an enzyme that is necessary for the *de novo* biosynthesis pathway of the nucleotide-activated sugar. In the de novo biosynthesis pathway, the nucleotide-activated sugar is synthesized from a "simple" carbon source such as glucose, sucrose, fructose, glycerol or the like.

The gene encoding the enzyme that is necessary for the *de novo* biosynthesis pathway of the nucleotide activated sugar can be endogenous to the bacterial cell or they can be introduced from exogenous sources into the cell to be transcribed and translated in a functional manner. Functional biosynthesis of proteins encoded by endogenous genes can be modified by genetically engineering the bacterial cell. For example, alteration of the expression of the endogenous gene may be achieved by modifying the gene's transcriptional promotor, by modifying the gene's ribosome binding site and/or by modifying the codon usage of the gene's protein coding region. In addition, the protein-coding nucleotide sequence of the gene may be modified such that the activity and/or the specificity of the encoded enzyme becomes favorable for the desired biosynthesis pathway. A person skilled in the art would know which gene(s) need to be expressed in the genetically modified organism for *de novo* synthesis of nucleotide activated sugar donor molecules.

In embodiments wherein a salvage pathway is used for the biosynthesis of the nucleotide-activated sugar donor substrate, the bacterial cell comprises an enzyme which catalyzes the linkage of a nucleotide and a monosaccharide. An example for such an enzyme is the bifunctional fucokinase/L-fucose-1-phosphate-guanylyltrans-ferase FKP from *Bacteroides fragilis* which catalyzes a kinase reaction and a pyrophosphatase reaction to form GDP-fucose. A further example for such an enzyme is the *N*-acetylneuraminate cytidyltransferase NeuA from *Neisseria meningitidis* which forming CMP-neuraminic acid.

A monosaccharide that is used as a substrate for the formation of the nucleotide-activated sugar in the salvage pathway may be internalized by the bacterial cell utilizing a transport protein. Examples of such transporter proteins are the fucose permease FucP and the sialic acid transporter NanT from *E. coli.* The transport protein may be encoded by and expressed from an endogenous gene or may be encoded by and expressed from a recombinant gene.

In certain embodiments, at least one endogenous gene of the bacterial cell which encodes an enzyme or a protein that prevents or interferes with the biosynthesis of the nucleotide-activated sugar and/or the oligosaccharide of interest is deleted or functionally inactivated.

The genetically engineered gram-negative bacterial cell possesses a saccharide transporter in its inner membrane. The saccharide transporter translocates the oligosaccharide of interest from the cytoplasm of the bacterial cell across its inner membrane into the periplasmic space.

A saccharide transporter mediating the translocation of a carbohydrate, e.g. the oligosaccharide of interest, across the inner membrane may consist of a single polypeptide or may consist of multiple polypeptides (each one considered being a subunit) which forming a homomeric or heteromeric complex that translocates the carbohydrate across the inner membrane.

In some embodiments, the saccharide transporter is selected from the group of transporters as set forth in Table 1. In additional and/or alternative embodiments, the saccharide transporter is a member of the major facilitator superfamily (MFS), a member of the sugar:cation symporter family, a member of the nucleoside specific transporter family, a member of the ATP binding cassette superfamily, or a member of the phosphotransferase system family.

In certain embodiments, the saccharide transporter is encoded by and expressed from a functional recombinant gene. Hence, in such embodiments, the gram-negative bacterial cell comprises a functional gene or an operon which encodes the saccharide transporter or at least one subunit of a saccharide transporter complex which facilitating the translocation of the intracellularly produced oligosaccharide of interest across the inner membrane of the bacterial cell into its periplasm.

The term "operon" as used herein refers to a nucleotide sequence comprising two or more protein coding sequences that are transcribed from the same regulatory element for mediating and/or controlling expression in the bacterial cell.

The term "functional gene" as used herein, refers to a nucleic acid molecule comprising a nucleotide sequence which encodes a protein or polypeptide, and which also contains regulatory sequences operably linked to said protein-coding nucleotide sequence (open reading frame) such that the nucleotide sequence which encodes the protein or polypeptide can be expressed in/by the cell bearing said functional gene. Thus, when cultured at conditions that are permissive for the expression of the functional gene, said functional gene is expressed, and the cell expressing said functional gene typically comprises the protein or polypeptide that is encoded by the protein coding region of the functional gene. As used herein, the terms "nucleic acid" and "polynucleotide" refer to a deoxyribonucleotide or ribonucleotide polymer in either single- or double-stranded form, and unless otherwise limited, encompasses known analogues of natural nucleotides that hybridize to nucleic acids in a manner similar to naturally occurring nucleotides.

The term "deregulated" as used herein with respect to endogenous genes refers to an altered expression of the protein coding region of said endogenous gene as compared to the expression of the gene in the native progenitor of the genetically engineered bacterial cell. Expression of a gene can be deregulated by different means known to the skilled artisan. Examples of deregulating the expression of a gene include alterations of the native nucleotide sequence of the gene's promoter, alteration of the native nucleotide sequence of the gene's ribosomal binding site. The term "deregulated" comprises an increases expression of a gene as well as a decreased or impaired expression of the gene.

The term "operably linked" as used herein, shall mean a functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence. Accordingly, the term "Promoter" designates DNA sequences which usually "precede" a gene in a DNA polymer and provide a site for initiation of the transcription into mRNA. "Regulator" DNA sequences, also usually "upstream" of (i.e., preceding) a gene in a given DNA polymer, bind proteins that determine the frequency (or rate) of transcriptional initiation. Collectively referred to as "promoter/regulator" or "control" DNA sequence, these sequences which precede a selected gene (or series of genes) in a functional DNA polymer cooperate to determine whether the transcription (and eventual expression) of a gene will occur. DNA sequences which "follow" a gene in a DNA polymer and provide a signal for termination of the transcription into mRNA are referred to as transcription "terminator" sequences.

The term "recombinant", as used herein indicates that a polynucleotide, such as a gene or operon, has been created by means of genetic engineering. Hence, a recombinant gene, operon or polynucleotide is not naturally occurring in cells of the same species as the gram-negative bacterial cell for the production of the oligosaccharide of interest. The recombinant polynucleotide may contain a heterologous nucleotide sequence, or expresses a peptide or protein encoded by a heterologous nucleotide sequence (i.e., a nucleotide sequence that is "foreign to said bacterial cell"). Recombinant bacterial cells can contain genes that are not found in its native (non-recombinant) predecessor. Recombinant cells can also contain variants of genes that are found in the recombinant cell's native predecessor, wherein the genes were modified and re-introduced into the cell by technical means. The term "recombinant" also encompasses bacterial cells that contain a nucleotide sequence that is endogenous to the bacterial cell and has been modified without removing the nucleic acid molecule bearing said nucleotide sequence from the bacterial cell. Such modifications include those obtained by gene replacement, site-specific mutation, and related techniques. Accordingly, a "recombinant polypeptide" is one which has been produced by a recombinant cell.

A "heterologous nucleotide sequence" or a "heterologous nucleic acid", as used herein, is one that originates from a source foreign to the particular host cell (e.g. from a different species), or, if from the same source, is modified from its original form. Thus, a heterologous nucleotide sequence may be a nucleotide sequence wherein a heterologous protein coding nucleotide sequence being operably linked to a promoter, wherein the protein coding nucleotide sequence and the nucleotide sequence of the promoter were obtained from different source organisms, or, if from the same source organism, either the protein coding nucleotide sequence or the promoter has been modified from its original form. The heterologous nucleotide sequence may be stably introduced into the genome of the bacterial cell, e.g. by transposition, transfection, transformation, conjugation or transduction. The techniques that may be applied will depend on the bacterial cell and the specificities of the nucleic acid molecule to be introduced into the bacterial cell. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989). Accordingly, a "genetically engineered bacterial cell" or "genetically engineered microbial cell" is understood as a bacterial cell which has been transformed or transfected, or is capable of transformation or transfection by an exogenous polynucleotide sequence. Thus, the nucleotide sequences as used in the invention, may, e.g., be comprised in a vector which is to be stably transformed/transfected or otherwise introduced into host microorganism cells. A great variety of expression systems can be used to produce polypeptides. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and to synthesize a polypeptide in a bacterial host cell may be used for expression in this regard. The appropriate nucleotide sequence may be inserted into the expression system by any of a variety of well-known and routine techniques, such as, for example, those set forth in Sambrook et al., supra. Preferably, polynucleotides containing the recombinant nucleotide sequence are stably introduced into the genome of the gram-negative bacterial cell. Genomic integration can be achieved by recombination or transposition.

The functional gene or functional genes encoding the transporter or the transporter complex facilitating the transport of the oligosaccharide of interest across the inner membrane can either be endogenous or exogenous to the genetically engineered bacterial cell.

In one embodiment of the invention the transporter gene is endogenous to the genetically engineered bacterial cell. Functional elements effecting synthesis of the protein that is encoded by the open reading frame of the transporter gene may have been modified such that the level of transcription of the open reading frame or translation of the mRNA leading to synthesis of the transporter protein is different from that naturally found in the predecessor cell. Modification of the functional elements can either increase the transcription and/or translation level or reduce the transcription and/or translation level compared to the unmodified state. Modifications can be single nucleotide modifications or exchange of complete functional elements. In either case the non-natural level of transcription and/or translation is optimized for the transport of the intracellular oligosaccharide of interest across the inner membrane.

In another embodiment the gene or genes encoding the transporter or transporter complex are exogenous to the genetically engineered bacterial cell. In such embodiments, the protein coding region is operably linked to naturally occurring or artificial functional elements such as promoters or ribosome binding sites in a way that the transcription level and the translation level of the transporter is optimized for the transport of the oligosaccharide of interest across the inner membrane.

Transcription of the gene(s) encoding the saccharide transporter or the subunits of the saccharide transporter can either be constitutive or regulated. A person skilled in the art would know how to choose and use a promoter and/or a promoter in combination with a further transcriptional regulator to achieve optimal expression of the gene(s) encoding the saccharide transporter.

In embodiments wherein the gene(s) encoding the saccharide transporter is exogenous to the bacterial cell for the production of the oligosaccharide of interest, the nucleotide sequence of the gene(s) may be modified as compared to the naturally occurring nucleotide sequence, but may encode a polypeptide possessing an unaltered amino acid sequence.

The saccharide transporter for translocation of the oligosaccharide of interest across the inner membrane can have its native amino acid sequence, i.e. the amino acid sequence that is naturally found. Alternatively, the amino acid sequence of the saccharide transporter can be modified as compared to its native amino acid sequence such that the resulting variant has enhanced activity with respect to the translocation of the oligosaccharide of interest and/or may have other beneficial characteristics, e.g. enhanced protein stability or more stable integration into the inner membrane.

In particular embodiments, the nucleotide sequence encoding the saccharide transport protein(s) is artificial and accordingly, the amino acid sequence of the resulting protein(s) acting as transporter to export the oligosaccharide of interest from the cytoplasm into the periplasm is as such not found in nature.

The term "variant(s)" as used herein, refers to a polynucleotide or polypeptide that differs from a reference polynucleotide or polypeptide respectively, but retains the essential (enzymatic) properties of the reference polynucleotide or polypeptide. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to the persons skilled in the art.

Within the scope of the present invention, also nucleic acid/polynucleotide and polypeptide polymorphic variants, alleles, mutants, and interspecies homologs are comprised by those terms, that have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of at least about 25, 50, 100, 200, 500, 1000, or more amino acids, to a polypeptide encoded by a wildtype protein.

Accordingly, a "functional variant" of any of the genes/proteins disclosed therein, is meant to designate sequence variants of the genes/proteins still retaining the same or somewhat lesser activity of the gene or protein the respective fragment is derived from.

Suitable saccharide transporters and/or their genes are provided in Table 1.

**Table 1: Saccharide transporters and their genes for translocation of an oligosaccharide across the inner membrane upon expression in a gram-negative bacterial cell.**

| **No.** | **Name** | **Organism** | **Accession No.** |
|---|---|---|---|
| TP01 | YabM | *Bacillus amyloliquefaciens* | AFJ60154 |
| TP02 | YabM | *Erwinia pyrifoliae* | CAY73138 |
| TP03 | SET fam | *Serratia proteamaculans* | ABV39846 |
| TP06 | YabM | *Staphylococcus carnosus* | CAL27067 |
| TP07 | YabM | *Yersinia pseudotuberculosis* | ACC87677 |
| TP08 | SET fam | *Deinococcus radiodurans* | AAF10890 |
| TP09 | MFS fam | *Yersinia bercovieri* | ZP_04626686 |
| TP10 | SET A | *Yersinia pestis* | AEL71168 |
| TP11 | ProP | *Mannheimia succiniciproducens* | AAU37785 |
| TP12 | SET fam | *Aeromonas caviae* | ZP_08519960 |
| TP13 | MDE fam | *Yersinia enterocolitica* | CAL11245 |
| TP14 | SET A | *Enterobacter cloacae* | EIM37633 |
| TP15 | MdfA | *Hafnia alvei* | ZP_09376236 |
| TP16 | MdfA | *Yersinia pseudotuberculosis* | ABS48940 |
| TP17 | RND fam | *E. coli* MG1655 | AAC75523 |
| TP18 | MFS fam | *E. coli* MG1655 | AAC75243 |
| TP19 | SET fam | *E. coli* MG1655 | AAC73929 |
| TP20 | YdeA | *E. coli* MG1655 | AAC74601 |
| TP21 | MDE fam | *E. coli* | NP_418129 |
| TP22 | EmrD | *E. coli* | NP_415123 |
| TP23 | Pgal | *E. coli* | NP_417418 |
| TP24 | MdtD | *E. coli* | NP_416581 |
| TP25 | MdtG | *E. coli* | NP_415571 |
| TP26 | MdtH | *E. coli* | NP_415583 |
| TP27 | MdtK | *E. coli* | YP_025307 |
| TP28 | MdtL | *E. coli* | NP_418166 |
| TP29 | MdtM | *E. coli* | NP_418757 |
| TP31 | Nepl | *E. coli* | NP_418118 |
| TP32 | YajR | *E. coli* | YP_488719 |
| TP33 | YcaD | *E. coli* | YP_489170 |
| TP34 | YdeE | *E. coli* | NP_416051 |
| TP35 | YdhC | *E. coli* | YP_025306 |
| TP36 | YdhP | *E. coli* | NP_416174 |
| TP37 | YepQ | *E. coli* | NP_416342 |
| TP38 | YfcJ | *E. coli* | NP_416825 |
| TP39 | YgcS | *E. coli* | NP_417251 |
| TP40 | YhhS | *E. coli* | NP_417930 |
| TP41 | yjhB | *E. coli* | NP_418699 |
| TP42 | YnfM | *E. coli* | NP_416113 |
| TP46 | LmrC | *Streptomyces lincolnensis* | EU124663 |
| TP47 | melB | *Klebsiella pneumoniae* | AAA25067 |
| TP48 | RafP | *Pediococcus pentosaceus* | CAA83664 |
| TP49 | SetA | *Shigella boydii* | YP_001878882 |
| TP50 | SetA | *Cronobacter turicensis* | YP_003209066 |
| TP51 | NorM | *Vibrio paraheamolyticus* | BAC59742 |
| TP52 | YabM | *Shigella boydii* | YP_406621 |
| TP53 | MFS fam | *Citrobacter koseri* | YP_001454829 |
| TP54 | proP2 | *Haemophilus parainfluenzae* | EGC72107 |
| TP55 | SET fam | *Pectobacterium carotovorum* | ZP_03829909 |
| TP56 | YdeE | *Gallibacterium anatis* | YP_004420051 |
| TP57 | ProP | *Actinobacillus suis* | YP_006817167 |
| TP58 | ChvA | *Agrobacterium tumefaciens* | AAA22083 |
| TP62 | LmrA | *Lactococcus lactis* | L116532 |
| TP63 | LndW | *Streptomyces globisporus* | ADK23796 |
| TP65 | setL | *Lactococcus lactis* | WP_023189943 |
| TP67 | SET fam | *Escherichia vulneris* NBRC 102420 | GAL60130 |
| TP68 | MFS fam | *Serratia fonticola* | WP_024910347 |
| TP69 | MFS fam | *Hafnia alvei* DSM 30099 | KID04082 |
| TP70 | MFS fam | *Cedecea neteri* M006 | AIR60762 |
| TP71 | MFS fam | *Erwinia billingiae* Eb661 | YP_003740094 |
| TP72 | SET fam | *Pseudomonas sp.* MT-1 | BAP78849 |
| TP73 | SET fam | *Beauveria bassiana* D1-5 | KGQ13398 |
| TP74 | MFS fam | *Pseudomonas syringae pv. syringae* SM | WP_003421619.1 |

The gram-negative bacterial cell possesses a porin in its outer membrane for the translocation of the oligosaccharide of interest from the periplasmic space across the outer membrane into the culture medium.

Porins that mediate the translocation of the oligosaccharide of interest across the outer membrane of the bacterial cell are typically "beta barrel" proteins that form a pore in the outer membrane. Such porins are composed of beta sheets comprising i) non-polar amino acid residues facing outwards and thus allowing integration of the porin into the lipid layer of the outer membrane, and ii) polar residues facing the inner side of the "barrel" thereby creating an aqueous channel.

The functional gene encoding the porin facilitating the transport of the desired oligosaccharide across the outer membrane can either be endogenous or exogenous to the recombinant cell.

In one embodiment of the invention the porin gene is endogenous to the recombinant cell producing the desired oligosaccharide. Functional elements influencing transcription of the open reading frame can be modified such that the level of transcription of the open reading frame or translation of the mRNA leading to synthesis of the porin is different from that naturally found in the cell. Modification of the functional elements can either increase the transcription or translation level or reduce the transcription or translation level compared to the unmodified state. Modifications can be single nucleotide modifications or exchange of complete functional elements. In either case the non-natural level of transcription or translation is optimized for the transport of the oligosaccharide of interest across the outer membrane.

In another embodiment the gene encoding the porin is exogenous to the recombinant cell. The protein coding region is operably linked to natural or artificial functional elements such as promoters or ribosome binding sites in a way that the transcription level and the translation level of the porin is optimized for the transport of the oligosaccharide of interest across the outer membrane.

Transcription of the gene encoding the porin can either be constitutive or regulated. A person skilled in the art would know how to choose and use a promoter and/or a promoter plus transcriptional regulator to achieve optimal expression of the gene encoding the porin.

In case that the gene encoding the porin is exogenous to the oligosaccharide producing cell the nucleotide sequence of the gene can be modified compared to the natural nucleotide sequence but encoding the same amino acid sequence.

The porin for translocation of the oligosaccharide of interest across the outer membrane can have an amino acid sequence found in nature or the naturally occurring amino acid sequence can be modified such that the variant of the porin has improved characteristics with respect to the transport of the oligosaccharide.

In another embodiment the nucleotide sequence encoding the porin can be completely artificial and accordingly, the amino acid sequence of the resulting protein acting as carbohydrate porin is as such not found in nature.

In some embodiments, the porin is selected from the group consisting of the porins set forth in Table 2.

**Table 2: Porins and porin genes for translocating an oligosaccharide across the outer membrane of a gram-negative bacterial cell.**

| **No.** | **Name** | **Organism** | **Acc. No.** |
|---|---|---|---|
| por01 | ompF | *E. coli* | ACT42828.1 |
| por02 | ompG | *E. coli* | ACT43184.1 |
| por03 | ompG E15_174H | *E. coli* | ACT43184.1 Protein Variant |
| por04 | pga | *E. coli* | ACT42921.1 |
| por05 | rafY | *E. coli* | ACT45579.1 |
| por06 | algE | *Pseudomonas aeruginosa* | WP_003092105.1 |
| por07 | cymA | *Klebsiella oxytoca* | WP_004136082.1 |
| por08 | scrY | *Escherichia coli* | WP_000277208.1 |
| por09 | scrY | *Klebsiella pneumoniae* | AHM80897.1 |
| por10 | ompC | *E. coli* | ACT43965.1 |
| por11 | lamB | *E. coli* | ACT 45699.1 |
| por12 | ybfM - chiP | *Salmonella enterica* | WP_001258803.1 |
| por13 | ompF | *Salmonella enterica* | WP_000977713.1 |
| por14 | ompA | *Salmonella enterica* | WP_001674965.1 |
| por15 | ompN-ompC | *Salmonella enterica* | WP_000824309.1 |
| por16 | ompS | *Salmonella enterica* | WP_001080680.1 |
| por17 | lamB | *Salmonella enterica* | WP_000973645.1 |
| por18 | rafY | *E. coli Plasmid* | CAP07697 |
| por19 | kdgM | *Dickeya dadantii* | WP_013318071 |
| por20 | chiP | *E. coli* | WP_072721362 |
| por21 | chiP | *Vibrio campbellii* | WP_012127214 |
| por22 | ompA | *Pseudomonas aeruginosa* | WP_003087843 |
| por23 | porCa | *Rhodobacter capsulatus* | AAB41301 |
| por24 | Omp2a | *Brucella melitensis* | AXG24303 |
| por25 | Omp2b | *Brucella melitensis* | AMM72579 |
| por26 | momP | *Camphylobacter jejuni* | CAH56489 |
| por27 | oprB | *Burkholderia pseudomallei* | WP_155017773 |
| por28 | fomA | *Fusobacterium nucleatum* | EDK88768 |
| por29 | ompIP | *Fusobacterium nucleatum* | EFD81713 |
| por30 | somA | *Synechococcus elongatus* | AAC33403 |
| por31 | somB | *Synechococcus elongatus* | AAC33402 |
| por32 | pulD | *Klebsiella oxytoca* | WP_163524530 |
| por33 | ompW | *Salmonella enterica* | QJU91510 |
| por34 | Cpp1 | *Coxiella burnetii* | AAO89868 |

In certain embodiments, the porin is encoded by and expressed from a functional recombinant gene. Hence, in such embodiments, the gram-negative cell comprises a functional gene or operon which encodes the porin which translocates the oligosaccharide of interest across the outer membrane of the bacterial cell into the cell's environment.

The functional gene encoding the porin for translocating the oligosaccharide of interest across the outer membrane can either be endogenous or exogenous to the genetically engineered bacterial cell.

In one embodiment of the invention the porin gene is endogenous to the genetically engineered bacterial cell. Functional elements effecting synthesis of the protein that is encoded by the open reading frame of the porin gene may have been modified such that the level of transcription of the open reading frame or translation of the mRNA leading to synthesis of the porin protein is different from that naturally found in the predecessor cell. Modification of the functional elements can either increase the transcription and/or translation level or reduce the transcription and/or translation level compared to the unmodified state. Modifications can be single nucleotide modifications or exchange of complete functional elements. In either case the non-natural level of transcription and/or translation is optimized for the translocation of the oligosaccharide of interest across the outer membrane.

In another embodiment the gene encoding the porin is exogenous to the genetically engineered bacterial cell. In such embodiments, the protein coding region is operably linked to naturally occurring or artificial functional elements such as promoters or ribosome binding sites in a way that the transcription level and/or the translation level of the porin is optimized for the translocation of the oligosaccharide of interest across the outer membrane.

Transcription of the gene(s) encoding the porin can either be constitutive or regulated. A person skilled in the art would know how to choose and use a promoter and/or a promoter in combination with a further transcriptional regulator to achieve optimal expression of the gene(s) encoding the saccharide transporter.

In embodiments wherein the gene(s) encoding the porin is exogenous to the bacterial cell for the production of the oligosaccharide of interest, the nucleotide sequence of the gene(s) may be modified as compared to the naturally occurring nucleotide sequence, but may encode a polypeptide possessing an unaltered amino acid sequence.

The porin for translocation of the oligosaccharide of interest across the outer membrane can have its native amino acid sequence, i.e. the amino acid sequence that is naturally found. Alternatively, the amino acid sequence of the porin can be modified as compared to its native amino acid sequence such that the resulting variant has enhanced activity with respect to the translocation of the oligosaccharide of interest and/or may have other beneficial characteristics, e.g. enhanced protein stability or more stable integration into the outer membrane.

In particular embodiments, the nucleotide sequence encoding the porin is artificial and accordingly, the amino acid sequence of the resulting protein(s) for translocating the oligosaccharide of interest from the periplasmic space into the environment is as such not found in nature.

The genetically-engineered gram-negative bacterial cell is suitable and intended for the production of the oligosaccharide of interest in an industrial scale. The term "industrial scale" as used herein refers to an economically viable production of the oligosaccharide. In addition, the term "industrial scale" refers to a production volume of several kilogram of oligosaccharide, preferably several hundred kilograms, more preferably several tons of oligosaccharide being produced in a single batch fermentation or single fed batch fermentation step and/or in a single purification run.

The bacterial cell is capable of synthesizing the oligosaccharide of interest intracellularly if cultured in a medium and under conditions that are permissible for the bacterial cell to synthesize the oligosaccharide of interest. In some embodiments, the gram-negative cell is a genetically engineered bacterial cell that has been genetically engineered to possess all genes that are necessary for the intracellular synthesis of the oligosaccharide of interest as well as for the intracellular synthesis and/or import of precursor molecules for the intracellular synthesis of the oligosaccharide of interest.

In some embodiments, the oligosaccharide of interest is 3-fucosyllactose, the saccharide transporter is a sugar efflux transporter, and the porin is selected from the group consisting of OmpF, LamB and OmpC.

In some embodiments, the oligosaccharide is lacto-*N*-tetraose, the saccahride transpsorter is MdfA, and the prorin is selected from the group consisting of LamB, OmpC and OmpG.

In some embodiments, the oligosaccharide of interest is lacto-*N*-neotetraose, the saccharide transporter is selected from the group consistign of TP29, TP46, TP55, TP56 and TP72, and the porin is selected from the group consisting of por01, por08, por09, por10, and por15.

In some embodiments, the oligosaccharide of interest is 6'-sialyllactose, the saccharide transporter is selected from the group cosnsisting of MdfA, MdtM and MdtL, the porin is selected from the group consisting of OmpG, ScrY, OmpA, OmpF and CymA.

In one embodiment of the invention the oligosaccharide of interest is 6'-sialyllactose. The genetically engineered bacterium is equipped with genes necessary for the synthesis of the nucleotide activated sugar CMP-sialic acid and a functionally expressed 2,6-sialyltransferase. Secretion of the intracellularly produced and intracellularly accumulating charged trisaccharide into the fermentation medium is facilitated by expressing a gene encoding an outer membrane porin.

In another embodiment the genetically engineered cell producing 6'-sialyllactose expresses gene(s) coding for a transporter facilitating the translocation of the intracellular synthesized oligosaccharide across the cytoplasmic membrane plus a gene coding for an outer membrane porin. Thus, transport of the acid sugar from the cytoplasm into the periplasm and into the fermentation medium is enhanced as reflected by an increase of the concentration of the sialyllactose in the fermentation medium.

In a preferred embodiment the porin translocating the 6'-siallylactose across the outer membrane is selected from the group of oligosaccharide translocating porins or of porins belonging to the OmpA-OmpF-porine (OOP) family (www.tcdb.org).

In another embodiment the cell capable in producing 6'-sialyllactose comprises an exogenous gene encoding a transporter of the major facilitator (MFS) family that allows increased oligosaccharide production.

In a specific embodiment the 6'-sialyllactose synthesizing bacterium expresses as well the gene for an MFS transporter as a gene encoding porin.

In one embodiment the oligosaccharide of interest is the neutral tetrasaccharide lacto-*N*-tetraose. The gram-negative bacterium capable in producing lacto-*N-*tetraose expresses all genes necessary to synthesize the nucleotide activated sugars UDP-galactose and UDP-*N*-acetylglucosamine in a sufficient amount to convert lactose that is either provided to the cell or synthesized by the cell to lacto-*N*-triose II and in a second step to convert lacto-*N*-triose II into lacto-*N*-tetraose, with using a functional 1,3-*N*-acetylglucosaminyltransferase in the first step and a functional beta-1,3-galactosyltransferase in the second step.

In an embodiment the saccharide transporter for translocation of lacto-*N*-tetraose across the inner membrane is MdfA. Overexpression of the endogenous *mdfA* gene increases lacto-*N*-tetraose export into in the cell's surrounding environment.

In one embodiment the oligosaccharide of interest is lacto-*N*-neotetraose. The engineered bacterium capable in producing lacto-*N*-neotetraose expresses all genes necessary to synthesize the nucleotide activated sugars UDP-galactose and UDP-*N*-acetylglucosamine in a sufficient amount to convert lactose that is either provided to the cell or synthesized by the cell to lacto-*N*-triose II and in a second step to convert lacto-*N*-triose II into lacto-*N*-neotetraose, thereby using a functional 1,3-*N-*acetylglucosaminyl transferase in the first step and a functional beta-1,4-galactosyltransferase in the second step.

In some embodiments the translocation of 3-fucosyllactose from the cytoplasm into the cell's environment is enhanced by expression of a saccharide transporter gene and/or a porin gene. The 3-fucosyllactose producing cell beside the genes encoding the transporter and the porin expresses genes leading to sufficient amounts of intracellular GDP-fucose and synthesis of an active alpha-1,3-fucosyltransferase.

According to an aspect of the invention, provided is a method for the production of an oligosaccharide of interest, wherein , the method comprising the steps of
a) providing a genetically engineered gram-negative bacterial cell wherein the bacterial cell
   - possesses a cytoplasm, an inner membrane, an outer membrane and a periplasmic space between the inner membrane and the outer membrane,
   - is able to synthesize the oligosaccharide of interest intracellularly,
   - possesses
      (i) an oligosaccharide exporter in its inner membrane for the translocation of the oligosaccharide of interest from the cytoplasm across the inner membrane into the periplasmic space, and
      (ii) a porin in its outer membrane for the translocation of the oligosaccharide of interest from the periplasmic space across the outer membrane,
         wherein the oligosaccharide exporter and/or the porin is expressed from a recombinant gene
b) culturing the bacterial cell at conditions permissive for the bacterial cell to synthesize the oligosaccharide of interest intracellularly; and
c) retrieving the oligosaccharide of interest from the culture medium.

Culturing said genetically engineered bacterial cells means growing the cells in a culture medium containing a carbon source and an acceptor substrate. The carbon source can be selected from glycerol, glucose, fructose, sucrose, cellulose, glycogen, or a combination thereof.

Culturing the cells can either be conducted in a batch fermentation, in a fed-batch fermentation system or in a continues fermentation system. Preferably, the culturing process comprises a phase of exponential growth of the cells with the carbon source in excess and a second phase of cell growth limited by the carbon source.

Addition of the acceptor substrate can be performed i) once during the during the fermentation, ii) as bolus' several times during the fermentation or iii) in a continuous manner throughout the fermentation process.

Acceptor substrate that is not converted to the oligosaccharide product during the fermentation process can be degraded by the addition of enzymes exhibiting specific hydrolytic activity on the remaining acceptor oligosaccharide or/and on degradation products of the acceptor substrate. These enzymes can be added as pure enzymes, crude extracts of cells expressing said enzymes in high concentration or by addition of a second cell culture comprising cells, that synthesize such enzymes and secrete them into the culture medium. By hydrolytic degradation of the remaining acceptor substrate purification of the oligosaccharide product of interest from the fermentation is facilitated since oligosaccharides differing in only or two monosaccharide units are hardly to separate by technical means in a large scale.

The desired oligosaccharide product is secreted into the fermentation medium and the producing cells stay intact. The cells are separated from the fermentation medium at the end of the fermentation process by centrifugation or filtration and the desired oligosaccharide is purified from the supernatant.

To purify the oligosaccharide of interest from the fermentation medium different technical methods can be applied. These technics comprise but are not limited to: filtration, chromatography, dialyses, concentration by diafiltration or evaporation, and crystallization.

The purified oligosaccharide of interest can be stored and used in liquid form or as dried substance. To obtain the oligosaccharide of interest in a solid form it can be freeze-dried, spray-dried, granulated, crystallized, dried on a rolling-dryer or a bet-dryer.

Typically, genetically engineered bacterial cells are used for the fermentative production of oligosaccharides.
The genetically modified organism capable in producing the desired oligosaccharides is a gram-negative bacterium.

Oligosaccharides being produced by *E. coli are* considered GRAS or safe for human consumption.

In fermentative production of oligosaccharides, bacterial cells are cultured at conditions permissive for the production of the oligosaccharide. The oligosaccharide is retrieved from the cytoplasm of the bacterial cells or from the fermentation broth. Preferably, the oligosaccharide of interest is retrieved from the fermentation broth. Secretion of the oligosaccharide into the fermentation broth has the advantage of higher productivity due to less limitation in solubility and space availability, less steps necessary in recovery and therefore, higher recovery rates achieving a more effective process.

The present invention will be described with respect to particular embodiments and with reference to drawings, but the invention is not limited thereto but only by the claims. Furthermore, the terms first, second and the like in the description and in the claims are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are means A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

### Examples

### Example 1: LC/MS analytics

Identity of the oligosaccharides of interest was determined by multiple reaction monitoring (MRM) using an LC triple-quadrupole MS detection system (Shimadzu LCMS-8050). Precursor ions were selected and analyzed in quadrupole 1, followed by collision-induced fragmentation (CID) with argon and selection of fragment ions in quadrupole 3. Selected transitions and collision energies for intermediates and end-product metabolites are listed in Table 3. LNT, LNnT, 6'-SL and 3-FL in particle-free culture supernatants or cytoplasmic fractions were separated by high performance liquid chromatography (HPLC) using a Waters XBridge Amide HPLC column (3.5 µm, 2.1 Å 50 mm) for both neutral or acidic sugars. Neutral sugars were eluted with isocratic flow of H₂O with 0.1% (v/v) ammonium hydroxide. Before LC/MS analysis, the samples of neutral oligosaccharides were prepared by filtration (0.22 µm pore size) and cleared by solid-phase extraction on a Strata ABW ion exchange matrix (Phenomenex). 6'-sialyllactose containing samples were filtered through a 0.22 µm filter, heated for 5 min at 95°C and centrifuged for 5 min at 13.000 × *g.* HPLC separation of the acidic sugar was performed using gradient elution with buffer A consisting of 50% acetonitrile and buffer B consisting of 80% acetonitrile, both containing 10 mM ammonium acetate at pH 4.2. The HPLC system encompasses a Shimadzu Nexera X2 SIL-30AC_{MP} autosampler run at 8°C, a Shimadzu LC-20AD pump, and a Shimadzu CTO-20AC column oven running at 35°C for elution of neutral sugars and was adjusted to 50°C for elution of acidic sugars. For the analysis of neutral sugars 1 µl, for acidic sugar determination 0.5 µL was injected into the instrument. Flow rate was set 0.3 mL/min (neutral), 0.4 mL/min (acidic) corresponding to a run time of 5 min. Whereas neutral sugars were analyzed in negative ion mode, acidic sugars were examined in positive ion mode. The mass spectrometer was operated at unit resolution. Collision energy, Q1 and Q3 pre-bias were optimized for each analyte. Quantification methods were established using commercially available standards (Carbosynth and Elicityl).

**Table 3. List of diagnostic MRM transitions and parameters for the identification of LNT, LNnT, 3-FL and 6'-SL (CE = Collision energy) of the Shimadzu LCMS-8050 Triple Quadrupole (QQQ) Mass Analyzer**

| Metabolite | Mode | Precursor m/z | product m/z | Dwell time [msec] | Q1 Pre bias (V) | CE | Q3 Pre Bias (V) |
|---|---|---|---|---|---|---|---|
| 6'-SL | positive | 656,2 | 365,15 | 38 | -22 | -33 | -19 |
| | positive | 656,2 | 314,15 | 38 | -22 | -31 | -23 |
| | positive | 656,2 | 612,15 | 38 | -22 | -30 | -32 |
| LNT | negative | 706,2 | 202,1 | 9 | 32 | 22 | 19 |
| | negative | 706,2 | 142 | 9 | 32 | 31 | 23 |
| | negative | 706,2 | 382,1 | 9 | 32 | 17 | 26 |
| LNnT | negative | 706,1 | 179,2 | 9 | 34 | 29 | 17 |
| | negative | 706,1 | 263,25 | 9 | 34 | 21 | 17 |
| | negative | 706,1 | 382,3 | 9 | 34 | 17 | 26 |
| 3-FL | negative | 487 | 179,25 | 9 | 22 | 14 | 29 |
| | negative | 487 | 89 | 9 | 22 | 26 | 18 |
| | negative | 487 | 205,15 | 9 | 22 | 25 | 21 |

### Example 2: Culture medium

The culture medium used to grow bacterial cells for the production of the oligosaccharide or interest contained: 3 g/L KH₂PO₄, 12 g/L K₂HPO₄, 5 g/L (NH₄)SO₄, 0.3 g/L citric acid, 0.1 g/L NaCl, 2 g/L MgSO₄ × 7 H₂O and 0.015 g/L CaCl₂ × 6 H₂O, supplemented with 1 ml/L trace element solution (54.4 g/L ammonium ferric citrate, 9.8 g/L MnCl₂ × 4 H₂O, 1.6 g/L CoCL₂ × 6 H₂O, 1 g/L CuCl₂ × 2 H₂O, 1.9 g/L MnCl₂ × 4 H₂O, 1.1 g/L Na₂MoO₄ × 2 H₂O, 1.5 g/L Na₂SeO₃, 1.5 g/L NiSO₄ × 6 H₂O). The pH of the medium was adjusted to 7.0. Lactose was added to the medium in a concentration of between10 mM and 60 mM for intracellular synthesis of the oligosaccharide of interest.

### Example 3: Construction of recombinant plasmids encoding diverse transporters and porins, respectively

The plasmid pINT (as described in WO 2017/042382) was used as vector for cloning the coding sequences of various porins. Said vector comprises the ColE1 replicon, an ampicillin resistance gene and a gene encoding the tetracyclin repressor. The coding sequence of various porins were set under the constitutive Pₜₑₜ promoter. Transcription of the porin genes is terminated by the rrnB terminator. The porin is expressed concomitantly with a streptomycin resistance cassette allowing selection of cells that integrated the exogenous gene. The construct Pₜₑₜ-PORIN-rrnB-smRlox was fused with 600 bp flanks of *E. coli* K12 DNA homologous to the *asl* locus (interregion between *aslA* and *hem Y*) via overlap extension PCR and cloned into the pINT vector backbone via Hifi assembly (New England Biolabs, Massachusetts, USA).

The coding regions of various transporters were amplified by PCR. The pINT-asl-Pₜₑₜ-PORIN-smRlox-asl plasmids were amplified as vector backbones via inverse PCR - opening the plasmid at the tetracyclin promoter. The transporter genes were inserted via Hifi assembly in a way to set the transporter under a functional tetracyclin promoter and linking the coding regions of the transporter and the porin with a ribosomal binding site in-between. The coding sequences of various porins were fused with various transporter genes resulting in various plasmids each containing a gene encoding an oligosaccharide transporters and a gene encoding a porin under the transcriptional control of the tetracycline inducible promoter.

For insertion of the respective genes into the asl locus, the asl-Pₜₑₜ-TP-PORIN-smRlox-asl region was amplified from the respective plasmid and used for A-Red mediated homologous recombination. For random insertion of the Ptet-TP-PORIN-smRlox construct, this region was amplified by PCR with primers inserting the 19 bp recognition sites for the EZ-Tn5 Transposase (Lucigen, Middleton, USA). The *in vivo* EZ Tn5 Transposase-mediated integration of the constructs was performed according to the manufacturer's instructions. In analog, the EZ constructs of Pₜₑₜ-PORIN-smRlox were amplified by PCR from the pINT-asl-Pₜₑₜ-PORIN-smRlox-asl plasmids. Furthermore, the Ptet-TP-cmRfrt constructs were amplified by PCR.

### Example 4: Enhanced export of 3-FL by genetically engineered E. coli

A bacterial strain for the production of 3-FL was built by integration of an α1,3-fucosyltransferase gene (*fucT*) and the *E. coli lacY* gen encoding a lactose permease, a GDP-fucose pathway comprising genes encoding a phosphomannomutase *(manB),* a mannose-1-phosphate guanosyltransferase *(manC),* a GDP-mannose-4,6-dehydratase *(gmd)* and a GDP-fucose synthase *(wcaG)* as well as the oligosaccharide transporter gene *setA* (sugar efflux transporter A, as described in patent US 8,652,808) in an *E. coli* BL21 strain having the genes *lacZ* (β-galactosidase), ara (arabinose isomerase), *fuclK* (L-fucose isomerase, L-fuculokinase), and *wcaJ* (UDP-glucose:undecaprenyl phosphate glucose-1 phosphate transferase) deleted or inactivated.

The genetic element asl-Pₜₑₜ-TP-PORIN-smRlox-asl was integrated into the interregion between *aslA* (putative sulfatase) and *hemY* (protein HemY) via A-Red mediated homologous recombination, and clones were selected on 2YT medium containing streptomycin. Selected clones bearing recombinant genes encoding an oligosaccharide transporter and a porin were analyzed for 3-FL production in deep well plates. For 3-FL production, respective bacterial cells were inoculated in 200 µl mineral salts medium in microtiter plates. Cells were grown at 30 °C with 800 rpm shaking and under 80 % humidity. After 24 h incubation, 50 µl of the precultures were transferred to 400 µl of the same medium with 20 mM lactose in deep well plates. Cells were grown for 24 h under the same conditions. After harvesting the cells by centrifugation, the culture supernatant was heated for 5 min to 95 °C, centrifuged again and diluted in LCMS-grade water to determine the 3-FL concentration by LC-MS/MS.

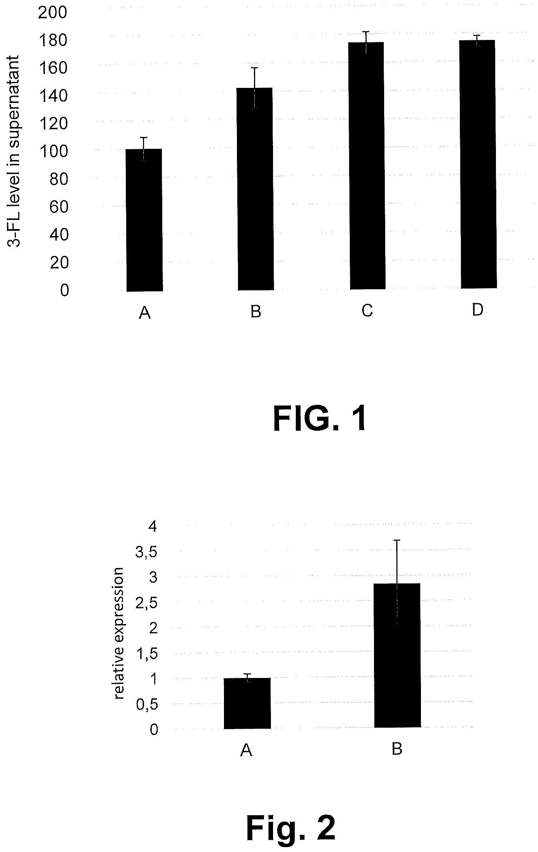

FIG. 1 illustrates that a recombinant *E. coli* strain expressing or overexpressing the *E. coli* porin OmpF (B) from a genomically integrated recombinant version of an *ompF gene* exhibit an improved export of 3-FL into the culture medium which contains a 42% increase of 3-FL level in the culture medium, as compared to the control (A) which is the parental *E. coli* strain not expressing a porin from a recombinant gene. *E. coli* strains bearing and expressing recombinant genes encoding the *Salmonella enterica* porins OmpF (C) or OmpC (D) display a further increased 3-FL export as the level of 3-FL in the culture supernatant is increased by 74 % or 75% respectively, as compared to the control (A).

Coexpression of a porin gene and a gene encoding an oligosaccharide transporter, each expressed from a recombinant gene being integrated into the genome of an *E. coli* 3-FL production strain, further enhances export of 3-FL as visualized in FIG. 2 by the approximately 2.5-fold amount of 3-FL found in the culture media of these transformants (B to I) as compared to the control (A) which is the precursor strain neither bearing a recombinant porin gene nor a recombinant oligosaccharide transporter gene.

### Example 5: Enhanced export of LNT by E. coli overexpressing an oligosaccharide transporter

A metabolically engineering *E. coli* BL21 (DE3) derivative (A) being capable to produce LNT was used for overexpression of the MdfA saccharide transporter. Therefore, the expression of *mdfA* was increased about 2.5-fold (B) via promotor mutagenesis using mismatch oligonucleotides (FIG. 2). The columns show the relative mdfA expression as quantified by the comparative ΔΔCt method. The calculated relative expression values were normalized to GapA and to control strain expression levels.

For expression analyses and determination of LNT amounts in the culture supernatant as well as intracellular respective clones were inoculated into 25 ml mineral salts medium in shaking flasks. Tests of respective clones were performed at least in 3 replicates. Cells were grown at 30°C with 120 rpm shaking and under 80% humidity. After 20 to 24 h incubation, the optical density of the preculture was determined to inoculate the main culture with an OD₆₀₀ of 0.05 in 25 ml of the same medium containing 10 mM lactose. Cells were grown again for 48 h under the same conditions. After harvesting the cells by centrifugation, the supernatant was heated for 5 min to 95°C centrifuged again and diluted into HPLC grade water prior to determination of the LNT concentration by LCMS. For determination of intracellular amounts 1.5 ml of the culture were centrifuged and the resulting pellet washed with 500 µl 4°C cold 0.9 % NaCl and centrifuged again. The resulting pellet was directly quenched with -20°C cold MeOH (50%). Cell debris was sedimented by centrifugation and the supernatant containing the intracellular metabolites used for respective dilutions and LCMS quantification.

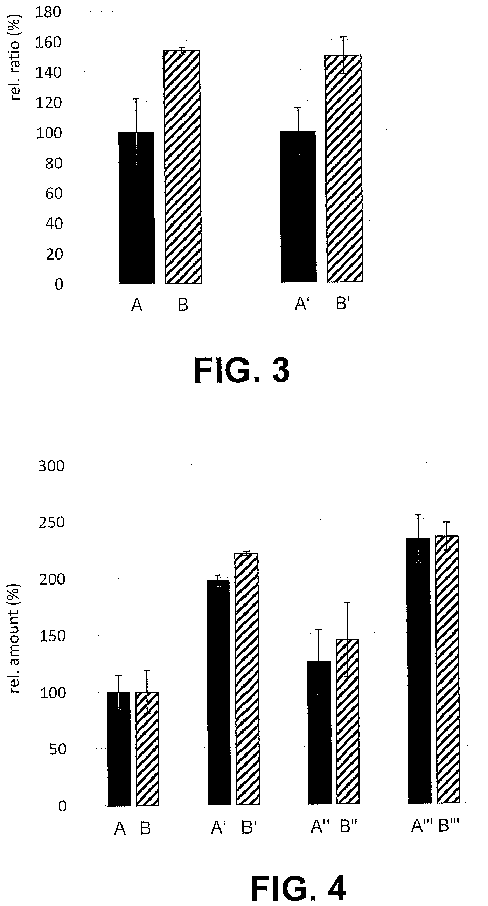

Overexpression of MdfA enhances export of LNT (FIG. 3, B') as well as of LNT-II (FIG.3, B) as revealed by elevated levels of LNT-II and LNT in the culture supernatant, as compared to the levels of LNT-II (FIG. 3, A) and LNT (FIG. 3, A') in the culture supernatant of the parental strains not overexpressing MdfA, whereas the intracellular amounts of LNT-II and LNT remained unaltered.

The effect of the facilitated LNT export was analyzed in lab scale fermentations for a duration of 96/100 hours. Fermentation in a 3-L-fermenter verifies an increased LNT production in an mdfA overexpressing strain as compared to the control strain of more than 60% at 96 hours fermentation time.

Expression of different porin genes in an *E. coli* strain that overexpresses mdfA increases LNT export from the bacterial cell as FIG. 4 displays. FIG. 4 illustrates that the export of LNT-II (A, A', A", A'") as well as of LNT (B, B', B", B'") is increased in strains that express *S*. *enterica* LamB (A', B'), *E. coli* BL21 OmpC (A", B") or the variant E15H, E174H (A"', B"') of the *E. coli* OmpG, as compared to the parental strain (A, B) which does not contain a recombinant porin gene for expression of any one of said three porins.

### Example 6: Enhanced export of LNnT by genetically engineered E. coli

A basic strain for producing LNT2 was generated by insertion of the 1,3-*N*-acetyl-glucosaminyl-transferase gene *IgtA* from *Neisseria meningitidis* in the araBA locus, the heterologous expression of *E. coli lacY* in an *E. coli* DH1 strain with the genes *wcaJ, lacZ, lacA, nagB, waaB, mdoH* deleted. The metabolic pathway to generate the nucleotide activated sugar UDP-galactose was enhanced by chromosomal overexpression of *E. coli pgm, galE and galU.* The 1,4-β-galactosyltransferase gene *lex-1* from *Aggregatibacter segnis* was set under a constitutive promoter, the rrnB terminator and a gentamycin resistance cassette. A 1,4-β-galactosyltransferase gene containing nucleotidesequence was amplified with primers adding recognition sites for the Tn5 EZ transposase allowing random *in vivo* transposon integration into *E. coli* yielding strains that produce LNnT.

For LNnT production, respective clones were inoculated in 200 µl mineral salts medium in microtiter plates. Cells were grown at 30 °C with 800 rpm shaking and under 80 % humidity. After 24 h incubation, 50 µl of the preculture were transferred to 400 µl of the same medium with 60 mM lactose. Cells were grown for 24 h under the same conditions. After harvesting the cells by centrifugation, the supernatant was heated for 5 min to 95 °C, centrifuged again and diluted in HPLC-graded water to determine the LNnT concentration by LC-MS/MS.

An *E. coli* LNnT-producing clone after EZ integration of lex-1 was used for further investigation of the export of LNnT. Via EZ Tn5 Transposase, various tetP-TP-PORIN-rrnB-smRlox constructs were randomly integrated into the genome of the LNnT-producing clone.

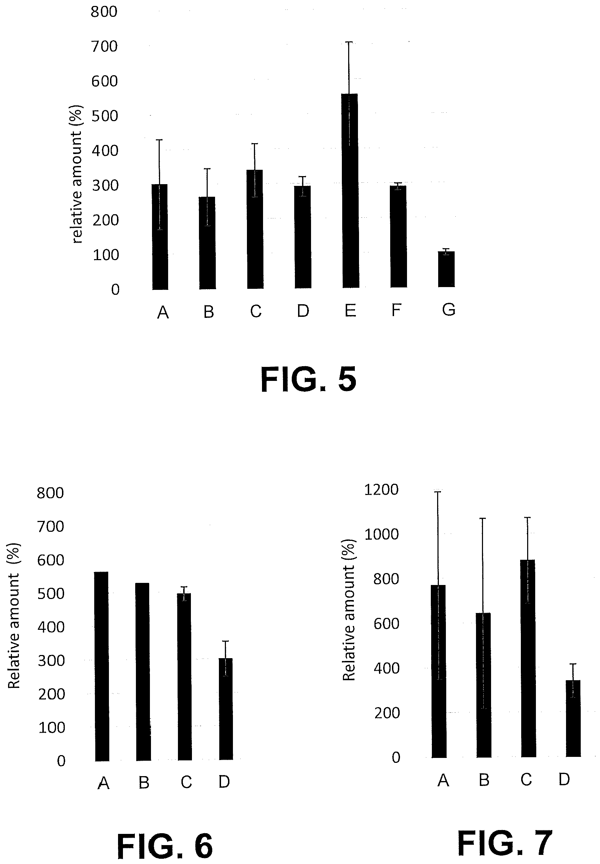

FIG. 5 illustrates that the relative amount of LNnT in the supernatant of cultures of individual *E. coli* strains expressing a saccharide transporter, a porin, or a porin and a saccharide transporter and display that the export is increased as compared to the parental strain used as control. In FIG 5, A designates an *E. coli* strain expressing saccharide transporter TP46, B designates an *E. coli* strain expressing saccharide transporter TP29 and porin por14, C designates an *E. coli* strain expressing porin por09, D designates an *E. coli* strain expressing porin por10, E designates an *E. coli* strain expressing porin por01, F designates an *E. coli* strain expressing porin por15, whereas G designates the parental *E. coli* strain neither expressing a saccharide transporter nor a porin from a recombinant gene.

FIG. 6 illustrates that the relative amount of LNnT in the supernatant of cultures of individual *E. coli* strains expressing a saccharide transporter, or a porin and a saccharide transporter, and displays that the export is increased as compared to the parental strain used as control. In FIG 6, A designates an *E. coli* strain expressing saccharide transporter TP46 and porin por08, B designates an *E. coli* strain expressing saccharide transporter TP46 and porin por09, C designates an *E. coli* strain expressing saccharide transporter TP46 and porin por10, whereas D designates an *E. coli* strain designates expressing saccharide transporter TP46 but no porin from a recombinant gene.

FIG. 7 illustrates that the relative amount of LNnT in the supernatant of cultures of individual *E. coli* strains expressing a porin or a porin and a saccharide transporter, and displays that the export is increased as compared to the parental strain used as control. In FIG 7, A designates an *E. coli* strain expressing saccharide transporter TP55 and porin por09, B designates an *E. coli* strain expressing saccharide transporter TP56 and porin por09, C designates an *E. coli* strain expressing saccharide transporter TP72 and porin por09, whereas D designates an *E*. *coli* strain designates expressing porin por09, but no saccharide transporter from a recombinant gene.

### Example 5: Enhanced export of 6'-SL by genetically engineered E. coli

A metabolically engineering *E. coli* BL21 (DE3) derivative capable of producing *N*-acetylneuraminic acid was further modified by genomic integration of a *neuA* gene and a *siaT* gene, encoding a CMP-sialic acid synthetase and an alpha-2,6-sialylltransferase respectively. Expression of both genes was under transcriptional control of constitutive promoters, yielding an *E*. *coli* strain that is able to synthesize 6'-SL intracellularly.

Genes coding for porin proteins were integrated into the genome of a clone from above-referenced *E*. *coli* strain by EZ-transposition. Therefore, the respective porin gene, preceded by the constitutive promotor Ptet and accompanied by the streptomycin resistance gene were amplified from vector pINT-asl-Porin-Smlox. For genomic integration of combinations of an oligosaccharide transporter gene with a porin gene, the respective genes were amplified from pINT-asl-Ptet-TP-PORIN-smRlox-asl and integrated into the genome of another clone for above-referenced *E. coli* strain. To this end, EZ-transposase (Lucigene, Middleton, USA) was used according to the manufacturer's description transforming electrocompetent *E*. *coli* cells with the transposomes. Cells containing chromosomal integrations were selected on 2YT medium containing streptomycin.

For production of 6'-siallylactose selected *E*. *coli* cells were inoculated in 200 µl mineral salts medium in microtiter plates. Tests of samples were performed in at least 3 replicates. The bacterial cells were grown at 30°C with 180 rpm shaking and under 80% humidity. After 20 to 24 h culturing, 50 µl of the preculture were transferred into 400 µl of mineral salts medium containing 40 mM lactose. Bacterial cells were grown again for 24 h at the same conditions. After harvesting the cells by centrifugation, the supernatant was heated for 5 min to 95°C centrifuged again and diluted into HPLC grade water prior to determine the 6'-sialyllactose concentration by LCMS.

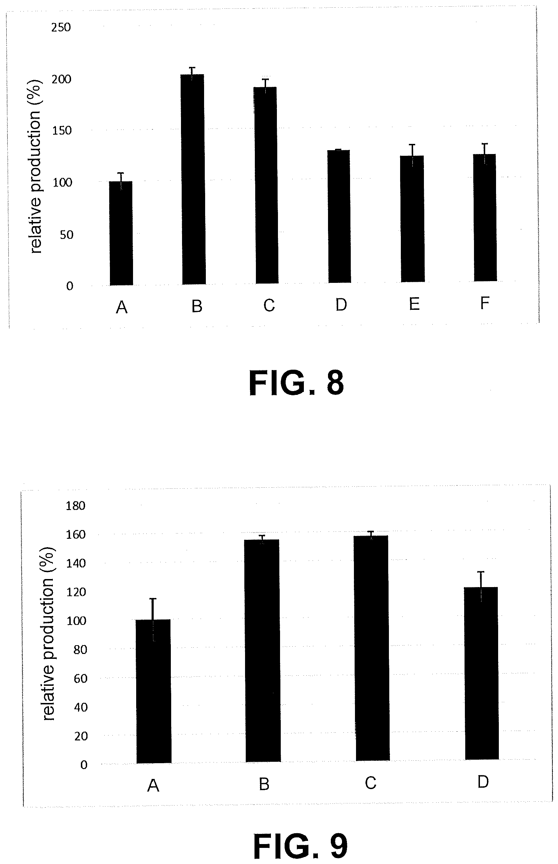

A two-fold increase of 6'-siallylactose amounts in the supernatant of cultures was achieved by genomic integration of the porin gene encoding an OmpG variant (FIG. 8, B) or the porin gene encoding ScrY of K. *pneumoniae* (FIG. 9, C). A 20-30% increased 6'-sialyllactose production was achieved by overexpressing ompA of S. *enterica* (FIG.8 D), OmpF of *E. coli* (FIG. 8, E) or CymA of *K. oxytoca* (Fig. 8, F) as compared to the parental E. coli strain (FIG. 8, A) that does not express a porin from a recombinant gene.

By concomitant expression of recombinant genes encoding a saccharide transporter and a porin in a 6'-SL producing *E. coli* stain increase 6'-sialyllactose production by 20 to 55 % as measured by relative amounts of 6'-SL in the culture supernatants. In FIG. 9, A designates the control strain that neither bear a recombinant porin gene nor a recombinant saccharide transporter gene, B designates the genetically engineered descendent bearing a recombinant mdfA gene and a recombinant K. *oxytoca camA* gene, C designates the genetically engineered descendent bearing a recombinant *mdtM* gene and a recombinant *E. coli ompF* gene, and D designates the genetically engineered descendent bearing a recombinant *mdtL* gene and a recombinant *ompG* gene from *E. coli.*

## Claims

1. A genetically-engineered gram-negative bacterial cell for the production of an oligosaccharide of interest, wherein the bacterial cell
- possesses a cytoplasm, an inner membrane, an outer membrane and a periplasmic space between the inner membrane and the outer membrane,
- is able to synthesize the oligosaccharide of interest intracellularly,
- possesses
(i) an oligosaccharide exporter in its inner membrane for the translocation of the oligosaccharide of interest from the cytoplasm across the inner membrane into the periplasmic space, and
(ii) a porin in its outer membrane for the translocation of the oligosaccharide of interest from the periplasmic space across the outer membrane,
wherein the oligosaccharide exporter and/or the porin is expressed from a recombinant gene or a deregulated endogenous gene.

2. The genetically-engineered bacterial cell according to claim 1, wherein the oligosaccharide of interest is a human milk oligosaccharide.

3. The genetically-engineered bacterial cell according to claim 2, wherein the human milk oligosaccharide is selected from the group consisting of neutral HMOs and sialylated HMOs, preferably from the group consisting of 2'-fucosyllactose, 3-fucosyllactose, 2',3-difucosyllactose, lacto-*N*-triose II, lacto-*N*-tetraose, lacto-*N*-neotetraose, lacto-*N*-fucopentaose I, lacto-*N-*neofucopentaose I, lacto-*N*-fucopentaose II, lacto-*N*-fucopentaose III, lacto-*N-*fucopentaose V, lacto-*N*-neofucopentaose V, lacto-*N*-difucohexaose I, lacto-*N-*difucohexaose II, 3'-galactosyllactose, 6'-galactosyllactose, lacto-*N*-hexaose, lacto-*N*-neohexaose, para-lacto-*N*-hexaose, para-lacto-*N*-neohexaose, difucosyl-lacto-*N*-neohexaose, 3'-sialyllactose, 6'-sialyllactose, lacto-*N-*sialylpentaose a, lacto-*N*-sialylpentaose b, lacto-*N*-sialylpentaose c, fucosyl-lacto-*N*-sialylpentaose a, fucosyl-lacto-N-sialylpentaose b, fucosyl-lacto-*N-*sialylpentaose c, disialyl-lacto-*N*-tetraose, disialyl-lacto-*N*-fucopentaose, disialyl-lacto-*N*-fucopentaose, 3-fucosyl-3'-sialyllactose, 3-fucosyl-6'-sialyllactose and lacto-*N*-neodifucohexaose I.

4. The genetically-engineered bacterial cell according any one of the preceding claims, wherein the oligosaccharide of interest is heterologously produced in the bacterial cell.

5. Use a gram-negative bacterial cell according to any one of claims 1 to 4 for the production of an oligosaccharide of interest.

6. A method for producing an oligosaccharide of interest, the method comprising the steps of
a) providing a genetically engineered gram-negative bacterial cell wherein the bacterial cell
- possesses a cytoplasm, an inner membrane, an outer membrane and a periplasmic space between the inner membrane and the outer membrane,
- is able to synthesize the oligosaccharide of interest intracellularly,
- possesses
(i) an oligosaccharide exporter in its inner membrane for the translocation of the oligosaccharide of interest from the cytoplasm across the inner membrane into the periplasmic space, and
(ii) a porin in its outer membrane for the translocation of the oligosaccharide of interest from the periplasmic space across the outer membrane,
wherein the oligosaccharide exporter and/or the porin is expressed from a recombinant gene
b) culturing the bacterial cell at conditions permissive for the bacterial cell to synthesize the oligosaccharide of interest intracellularly; and
c) retrieving the oligosaccharide of interest from the culture medium.

7. A method for enhancing the export of an oligosaccharide of interest from a gram-negative bacterial cell into a culture medium said bacterial cell is cultured in, wherein the bacterial cell has been genetically engineered to synthesize the oligosaccharide of interest intracellularly, the method comprising the step of expressing a saccharide transporter and/or a porin from a recombinant gene in said bacterial cell and/or by deregulating the expression of an endogenous saccharide transporter and/or porin gene.
